# EUROPEAN PATENT SPECIFICATION

(11) **EP 3 246 069 B1**
(45) Date of publication and mention of the grant of the patent: **02.01.2019**
(21) Application number: 17171031.2
(22) Date of filing: 15.05.2017
(51) Int. Cl.: A61N 5/06

(54) **IMPROVED MEDICAL DEVICE FOR LASER THERAPY**
VERBESSERTE MEDIZINISCHE VORRICHTUNG ZUR LASERTHERAPIE
DISPOSITIF MÉDICAL AMÉLIORÉ DE THÉRAPIE LASER

(30) Priority: 16.05.2016 IT UA20163480
(43) Date of publication of application: 22.11.2017
(73) Proprietor: Winform Medical Engineering S.r.l., 30027 San Dona' di Piave (Venezia) (IT)
(72) Inventor: PIZZOCHERO, Franco, 30027 San Dona' di Piave (Venezia) (IT)
(74) Representative: Piovesana, Paolo

(56) References cited:
- WO-A1-2013/052482
- US-A1- 2007 060 984
- US-A1- 2007 239 232
- US-A1- 2013 304 164

## Description

The present invention relates to an improved medical device for laser therapy.

As known, laser (which is the acronym of the expression "Light Amplification by Stimulated Emission of Radiation") is an emission source of a coherent light beam which is capable of producing energy in form of a light wave following a stimulated emission of radiations. In particular, the laser beam is generated by means of an amplification process of an energized atomic system, which constitutes the active means and which may be obtained with solid materials, in particular semiconductors, liquid or gaseous materials.

In the medical field, laser technology for therapeutic purposes is generally used to exploit the biological effects which are induced on the tissues of the human body by the energy generated by the laser light.

In particular, according to the wavelength used and by administering suitable energy doses, different interactions can be obtained with the tissues so as to generate a particularly accurate and targeted therapeutic action (antalgic, anti-inflammatory, etc.). More in detail, the laser light emission may be continuous or pulsed, and is characterized by a wavelength (λ), expressed in nanometers, which determines the absorption power by the tissues on which the laser light is applied.

Many and various laser therapy devices exist in the current art, but they are not fully satisfactory because they generally comprise a single laser source which always and only emits photons at a given wavelength. Furthermore, in the case of devices with multiple laser sources, the latter are controlled so as to emit the corresponding photons in sequential and distinct manner.

Not only, in such known devices, the power emitted by the laser source in form of photons is dissipated importantly inside the device itself, before reaching the tissue to be treated, and this implies a decrease of the therapeutic effects which can be obtained.

US2013/0304164 describes a portable device to be used in physiotherapy and/or surgery, which comprises a containment casing, in which one or more laser sources are housed, which are directly connected to an optical collimator, which combines the photons emitted by the laser sources before they enter into a single external flexible cable, which consists of an optical fiber and which is provided with an applicator handpiece at an end thereof. Such a solution is not fully satisfactory because it is rather complicated to position the optical collimator at the laser source outputs.

US2007/0060984 specifically describes a device which uses light as source to provide a precise stimulation on one or more nerve fibers. In particular, the device comprises a laser diode bar and a visible light laser source, which are associated, by means of one or more optical fibers, with an optical collimator, which is in turn operatively connected, by means of one or more optical fibers, to a tip provided with an emission optics. More in detail, in such a solution, the visible light source is exclusively provided as pointer and to provide information related to the state of a given function of the device, without having any therapeutic function or action on the tissues.

WO2013/052482 describes a system for photodynamic therapy, which comprises two laser sources, each of which is connected in output to a corresponding leg of a bifurcated optical fiber. In particular, the cores of the two legs of the bifurcated optical fiber are then fused together at a fusion point so as to form a single core having a diameter of 200-400 micron. Such a solution is not satisfactory because it does not provide any optical collimator and furthermore the joining of the optical fibers, being made by fusing, is of the permanent type and this does not allow any uncoupling between the fibers for the purposes of their maintenance (e.g. to replace a damaged fiber).

US2007/0239232 describes a laser therapy device, in which the light source is associated by means of optical fibers with a bandage to be applied on the tissues to be treated, which is made of flexible material and which is provided with extraction means to address the light output from the bandage itself towards said tissues.

It is the object of the present invention to make a device which overcomes the drawbacks of the traditional devices and which is improving and/or alternative to them.

It is another object of the invention to make a device which is simple, quick and easy to assemble.

It is another object of the invention to make a device which displays an alternative characterization, in both construction and functional terms, with respect to the traditional ones.

It is another object of the invention to make a device which is simple, quick and easy to obtain.

It is another object of the invention to make a device which provides action means to the operator to intervene, simultaneously on the same subject or in sequence on different subjects, on pathologies validated for treatment with laser therapy.

It is another object of the invention to make a high quality automatic device, which is reliable, non-invasive, energy-efficient and safe for the operator and for the patient.

It is another object of the invention to make a device which is quick and easy to maintain.

All these objects, taken individually or in any combination thereof, and others which will be apparent from the following description, are achieved, according to the invention, by an improved medical device for laser therapy having the features indicated in claim 1.

The present invention is further explained by means of a preferred embodiment given by way of non-limiting practical example only with reference to the accompanying drawings, in which:
Figure 1 shows a perspective view of the device according to the invention,
Figure 2 diagrammatically shows a detail thereof,

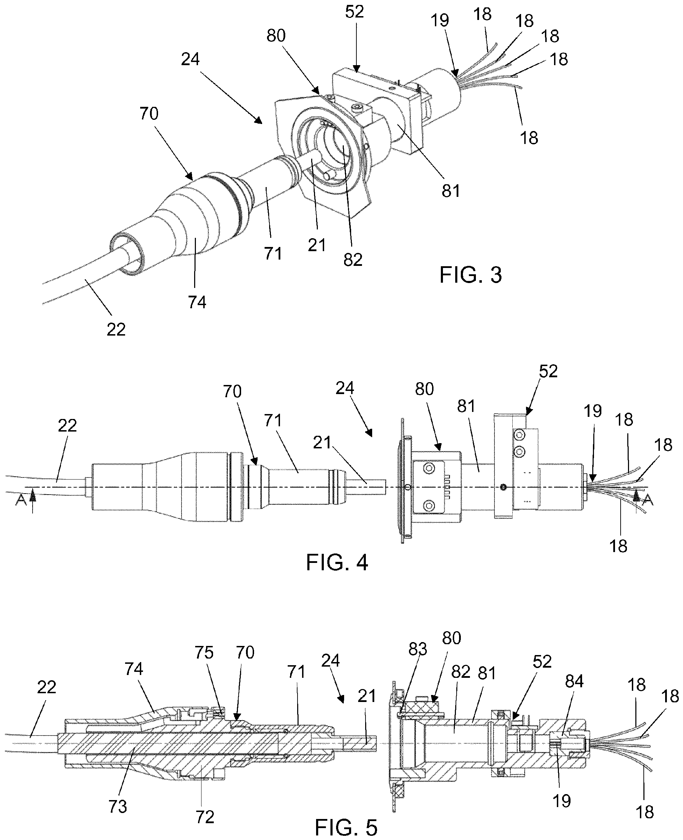

Figure 3 shows a perspective view of the mechanical connection provided in the device according to the invention, in a condition in which the two portions of said connection are mutually uncoupled,
Figure 4 shows a side view of the mechanical connection in figure 3,
Figure 5 shows section A-A in figure 4,

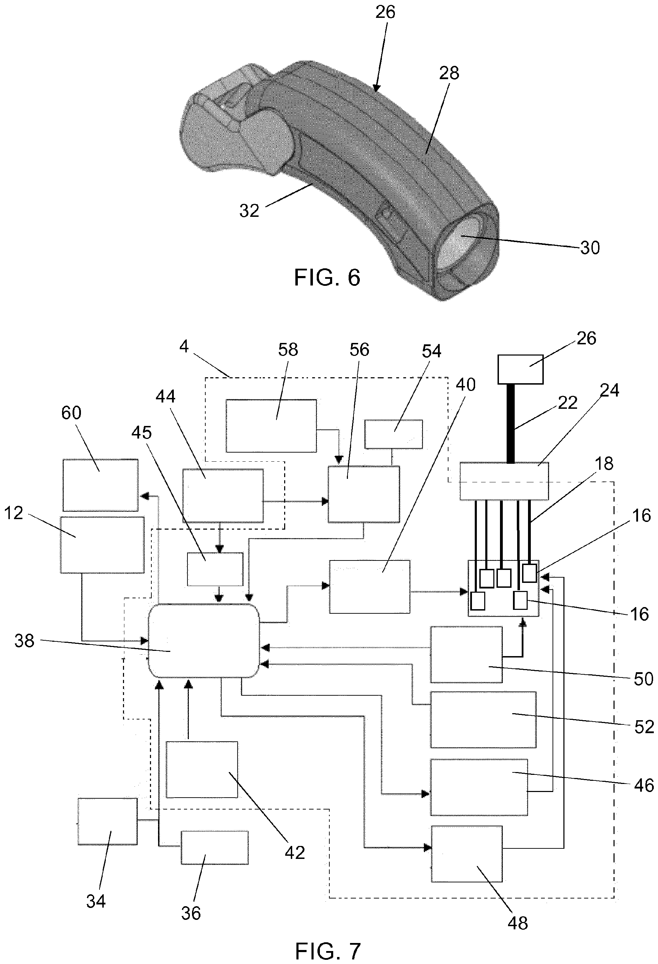

Figure 6 shows a perspective view of the applicator handpiece only,
Figure 7 shows the functional connections between the components of the device in a block chart.

As shown in the figures, the improved medical device 2 for laser therapy according to the invention comprises a containment casing 4, which is preferably associated with a support 6 provided with wheels 8 or other traditional means for easily moving the device itself in the work space.

Advantageously, the containment casing 4 is made of painted PVC and anodized aluminum in order to appropriately make the device highly biocompatible.

The device also comprises a user interface 10, which is externally associated with the containment casing 4 and is provided with a viewing screen 12 and a control panel provided with input means 14, which can be operatively activated by the operator. For example, the user interface 12 may comprise a touchscreen and/or a viewing monitor associated with a keypad/panel and/or a handpiece 14 associated with a rotary encoder.

Two, preferably four or five, laser sources 16 are housed inside the containment casing 4.

In particular, the laser sources 16 are of the light emitting semiconductor diode (LED) type, i.e. comprise a semiconductor material, which is doped with impurities such to form a positive-negative junction (the so-called "P-N junction") so that it emits photons at a specific wavelength when it is crossed by current.

More in detail, the diodes of the laser sources 16 are made of one or more semiconductor materials which are appropriately chosen and/or doped so that the diodes emit photons having all mutually different wavelengths, comprised in the red and infrared band, that is, substantially comprised between 550 nm and 1075 nm, and preferably comprised between about 625 nm and 1064 nm.

Furthermore, the LED diodes of the laser sources 16 are configured so that each one emits a power substantially comprised between 500 mW and 15 W, and preferably comprised between approximately 5W and 10W.

Preferably, five LED diode laser sources 16 are provided, each of which is configured to emit photons having different wavelength from those of the other sources. More in detail, the laser sources 16 comprise:
- a first laser source, which emits photons at about 580-680 nm, preferably at about 625 nm, with power of about 100 mW-15 W, preferably of about 5 W,
- a second laser source, which emits photons at about 750-795 nm, preferably at about 750-785 nm, with power of about 500 mW-15 W, preferably of about 5 W,
- a third laser source, which emits photons at about 800-850 nm, preferably at about 810 nm, with power of about 500 mW-15 W, preferably of about 10 W,
- a fourth laser source, which emits photons at about 915-975 nm, preferably at about 975 nm, with power of about 500 mW-15 W, preferably of about 10 W,
- a fifth laser source, which emits photons at about 1064 nm, with power of about 500 mW-15 W, preferably of about 8W.

Preferably, the diode packages of the laser sources 16 is of the C-mount type. Preferably, the diode package of the second, third, fourth and fifth laser source are of the C-mount type, while the diode package of the first laser source is of the TO-18 type.

Preferably, the diodes of the laser sources 16 are made using aluminum gallium arsenide (GaAIAs), which is appropriately doped so as to emit photons with the aforesaid wavelengths, as semiconductor material.

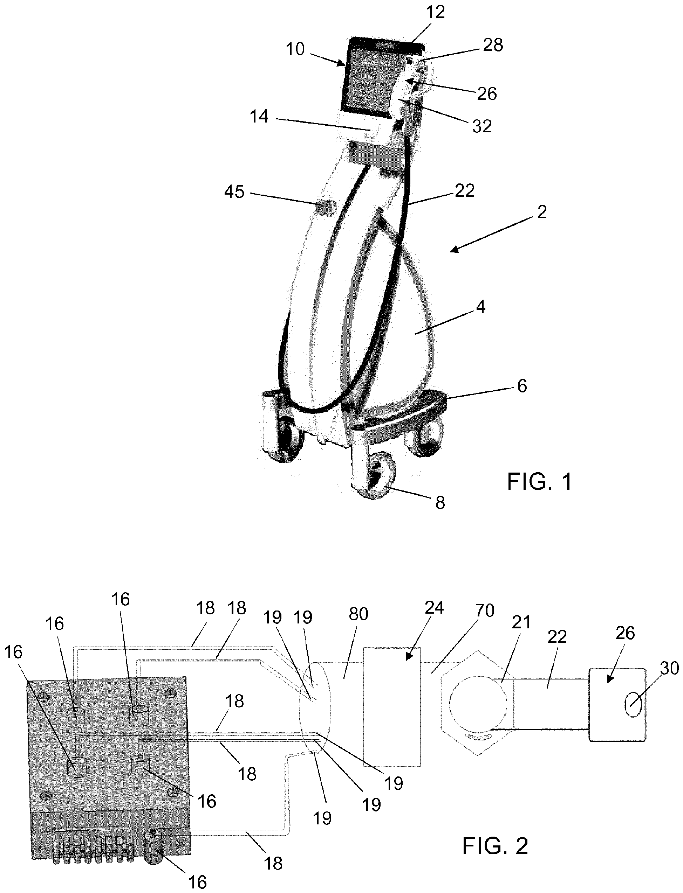

The photons emitted by each laser source 16 are emitted and conveyed directly into corresponding first optical fibers 18, also housed in the containment casing 4. In particular, each laser source 16 is associated with a first optical fiber 18 and, therefore, in the case of five laser sources 16 there are five optical fibers 18, one for each of the five sources (cf. fig. 2).

Preferably, the central core of each first optical fiber 18 has a diameter of about 0.12mm. Preferably, the first optical fibers 18 are made of silicone because they display less loss.

The device 2 further comprises a single second optical fiber 22, which is positioned, at least in part, inside the containment casing 4 and which receives the photons which exit from all the first optical fibers 18.

Advantageously, the second optical fiber 22, which is single for all laser sources 16, is made using the liquid phase method. Preferably, the second optical fiber 22 is flexible with a radius of curvature greater than about 14 mm, and a cylindrical core having a diameter of about 2-10 mm, preferably of about 8 mm.

Device 2 further comprises a mechanical connection 24 to connect the output terminals 19 of all the first optical fibers 18 - preferably made of silicone, each of which is associated with one of the laser sources 16 with the second optical fiber 22, which is preferably made using the liquid phase method and is positioned, at least in part, inside the containment housing 4. Preferably, the mechanical connection 24 defines a removable connection between the output terminals 19 of all first optical fibers 18 and the input end 21 of the second optical fiber 22 so that all the photons, which are emitted by the laser source 16 and which have crossed the first optical fibers 18, enter into the second optical fiber 22.

In particular, each first optical fiber 18 is inserted with an input end thereof inside the corresponding structure of the laser source 16, while the other (output) terminal 19 is channeled, together with the output terminals 19 of all the other first optical fibers 18, inside the mechanical connection 24.

Advantageously, the mechanical connection 24 is configured so that, inside, the input end 21 of the second optical fiber 22 faces and/or is aligned with the end terminals 19 of all said first optical fibers 18.

Advantageously, the mechanical connection 24 is configured so that, inside, the input end 21 of the second optical fiber 22 is either in contact with or in close proximity to (e.g. at a distance substantially smaller than one millimeter) the output terminals 19 of all said first optical fibers 18.

Appropriately, the diameter of the cross section of the second optical fiber 22 is much greater than that of the first optical fibers 18. Preferably, the cross section area of the second optical fiber 22 is either substantially equal to or greater than the sum (set) of the cross section areas of all the first optical fibers 18.

In particular, the fact that the second optical fiber 22 has a cross section area greater than that defined by the set of all the first optical fibers 13 combined with the use of a mechanical connection 24 in which the output terminals 19 of all the first optical fibers 18 are aligned/facing and in contact with/close proximity to the input end 21 of the single second optical fiber 22 avoids the use of complicated and costly collimators with particular refraction and/or focusing optics and lenses.

Advantageously, at the input end 21 of the second optical fiber 22 there is a lens (not shown), which acts as diffuser for the photons which from the inner optical fibers 18 enter into the single second optical fiber 22.

Advantageously, the mechanical connection 24, which is preferably made of anodized aluminum, may be associated with the containment casing 4.

Preferably, the mechanical connection 24 comprises two portions, respectively 70 and 80, which are mutually separable and couplable by joining means, preferably snap-fitting, e.g. of the male-female type.

In particular, the male portion 70 comprises a protruding head 71, preferably pin-like, and a tubular body 72 provided inside with a crossing and locking seat 73 of the second optical fiber 22. Appropriately, the second optical fiber 22 is engaged inside the head 71 so that its input end 21 protrudes from the head itself. Appropriately, an external covering and/or gripping sleeve 74 is also provided.

Appropriately, further seats for electrical connection wires to appropriate electrical boards housed inside the containing casing 4 may be provided at the head 71 of the male portion 70 of the mechanical connection 24.

The female portion 80 comprises a substantially tubular leg 81 with a central cavity 82, which is appropriately shaped and sized for the stable, but removable engagement and coupling of the protruding head 71 of the male portion 70. Advantageously, such type of connection is manually made by the user, i.e. without using any tool.

Advantageously, the female portion 80 comprises a guiding and reference pin 83 cooperating with a corresponding hole 75 defined in the male portion 70 in order to facilitate their coupling.

All the terminals 19 of the first optical fibers 18 are inserted and locked in a terminal part of the tubular leg 81 of the female portion 80; preferably, the terminals 19 are fixed to the tubular leg 81 by means of appropriate glue/transparent resin in order to prevent them from being pulled out. Advantageously, all the terminals 19 of the first optical fibers 18 may be inserted and locked in a bushing 85, which in turn is removably associated within the tubular leg 81 of the female portion 80.

Advantageously, the insertion of the protruding head 71 of the male portion 70 inside the cavity 82 of the female portion 80 carries the input end 21 of the single second optical fiber 22 - which is associated with the head 71 - to face and be either in contact with or in close proximity to (i.e. at a distance substantially smaller than 1 mm) the output terminals 19 of all first optical fibers 18, which are associated with the female portion 80.

Appropriately, said configuration of the mechanical connection 24 which connects the output terminals 19 of the first optical fibers 18 to the input end of a single second optical fiber 22 has low and repeatable insertion losses, low reflected power, stability over time, reliability, is low-cost and above all avoids the use of particular refraction and focusing optics.

The second optical fiber 22 is associated, on the other end thereof, with an applicator handpiece 26. In particular, the latter comprises an articulated head 28 with a single opening 30 communicating with the second optical fiber 22 to allow the release of the photons emitted by the laser sources 16 at the tissue to be treated. Appropriately, the applicator handpiece 16 also comprises a grip 32 to allow the operator to appropriately direct the photons output from the single opening 30 obtained in the handpiece itself onto the tissues of the patient to be treated.

The power of the light emissions of the laser sources 16 detected at the mechanical connection 24 is comprised in a range of about 5-60 W, preferably about 38 W, while that detected at the opening 30 of the applicator handpiece 26, and is thus sent to the tissues to be treated, is comprised in a range of about 1-60 W, in particular is about 15 W.

Advantageously, the device 4 comprises means for activating/deactivating the laser sources 16 and/or for allowing/blocking the output of photons from the single opening 30 obtained in the applicable handpiece 26. Preferably, these means comprise a pedal switch 34 or a switch 36 defined at the grip 32 of the handpiece 26.

The device also comprises a control and processing unit 38, e.g. a microprocessor and/or a CPU, which is housed in the containing casing 4 and which manages the entire device 4, and in particular appropriately controls the laser sources 16. The control and processing unit 38 is also functionally connected to the user interface 10.

More in detail, each laser source 16 is associated with a current/voltage generator 40, which is activated/deactivated and/or appropriately controlled by the control and processing unit 38.

Preferably, each laser source 16 is independently controlled, by the control and processing unit 38, so as to emit a pulse laser light beam and, in particular, for this purpose a square wave signal with frequency substantially comprised between 1 and 20000 Hz and duty cycle of at least 50% is controlled.

More in detail, on the basis of preset treatment cycles stored in the control and processing unit 38 and/or on the basis of the controls set by the operator by acting on the user interface 10, the control and processing unit 38 singularly and independently controls the enabling/disabling of each laser source 16, as well as the correspondence of their optical emission power. In particular, in this fashion, the control and processing unit 38 controls the number of laser sources 16 to be activated simultaneously and/or to be activated automatically in sequential manner.

Appropriately, the control and processing program 38 is performed by means of a connection with an external processor. As mentioned, treatment cycles are stored in the control and processing unit 38, each of which has predetermined and independent operating parameters, in terms of number of laser sources activated at the same time, emission power, activation sequence, etc.

Appropriately, the device 2 also comprises means 42, managed by the microprocessor, to detect the current drawn by the laser sources 16 in order to vary their corresponding efficiency state in this manner.

The power supply voltage of the laser sources 16 for the microprocessor of the control and processing unit 38 and for the other electrical elements provided inside the device 2 is provided by means of a connection 44 to the power network, which is preferably at 220V. Appropriately, an appropriate emergency control 45 is also provided so that the control and processing unit 38 may interrupt the input of the electricity from the connection 44 to the power supply network into the device 2.

Advantageously, a cooling system, configured and controlled to ensure the appropriate working temperatures in the containment casing 4, is also housed inside the containment casing itself. In particular, the cooling system comprises a heat sink with air introduction and extraction fans 46, as well as a Peltier cell 48 and a temperature sensor 50 for each laser source 16.

More in detail, the temperature sensors 50 are provided to send signals corresponding to the temperature detected at each laser source 16 to the control and processing unit 38. Appropriately, the control and processing unit 38 is configured to control the cooling fans 46 and the corresponding Peltier cell 48 associated with the laser source 16 appropriately according to the basis of the received temperature signals.

Advantageously, the device 2 also comprises an acoustic indicator 60 (e.g. a buzzer), which is controlled by the control and processing unit 38 to produce a warning when the laser sources 16 are activated.

Advantageously, the device 2 also comprises means 52 for detecting whether the optical fiber 22 is correctly connected to the corresponding connector 24 associated to the containment casing 4. Such means 52 appropriately comprise a traditional sensor which is mounted on a housing provided in the female portion 80 of the connector 24 and which is adapted to detect the presence and/or the passage of photons in the connector itself. In particular, in case of correct insertion of the male portion 70, associated with the second optical fiber 22, within the female portion 80 of the mechanical connection 24, the photons output by the first optical fibers 18 enter directly into the second optical fiber 22 and thus the sensor 52 does not detect any presence and/or anomalous passage (i.e. dispersion/loss) of photons in the mechanical connection 24.

Such sensor 52 is appropriately connected with the control and processing unit 38 so that the latter controls the automatic deactivation of the laser sources 16 and the sending of a visual and/or acoustic warning to the operator, if the second optical fiber 22 is not correctly inserted in the corresponding connector 24.

Advantageously, the device 2 also comprises a battery 54, which is also housed in the containment casing 4. Preferably, the battery 54 is managed by an appropriately electronic board 56 for managing recharging and is associated with a transformer 58, preferably of lamellar type. Appropriately, the battery 56 is activated by the control and processing unit 38 when it detects a temporary interruption of the external network power supply so as to allow, also in this case, an appropriate cooling of the laser sources 16 housed in the chasing 4.

The device according to the invention differs with respect to traditional devices in that none of the prior art documents US2013/0304164, US2007/0060984 and WO2013/052482 either describes or shows a solution provided with a plurality of first distinct optical fibers - each of which is associated with a laser source for therapeutic purposes (thus excluding those which can be used as pointers or light indicators) - which are all mechanically and removably connected to one only second optical fiber. This is particularly advantageous because it defines a particularly efficient method, which is simple to make and easy to maintain for transmitting a plurality of therapeutic light emissions to an applicator handpiece.

Furthermore, it is clearly apparent from the above that the device according to the invention is more advantageous because:
- by simultaneously emitting from two to five light emissions (photons) of different wavelengths it makes it possible to combine the anti-inflammatory, anti-edemigenous and antalgic actions (deriving mainly from photons at about 1064 nm with particularly high emission frequency, of about 10000-20000 Hz) with the cellular mitosis and myelin sheath regeneration stimulation actions (mainly deriving from the photons at 650 nm, 750 nm and 810 nm),
- it provides a single instrument which can be used to intervene on a high number of pathologies, the treatment of which by means of laser therapy is clinically validated, to the operator/therapist,
- it is suited for the application of laser therapy in dermatology, physiatry, rheumatology, pain therapy, sports medicine, etc.,
- it is safe, biocompatible, simple and easy to use for the operator,
- it makes manufacturing cost saving by virtue of its construction simplicity, and in particular of the fact that it does not have any particular collimation optics,
- it is simple to maintain because, by being mechanical and uncoupleable, the joining of the optical fibers allows the targeted replacement of the single, possibly malfunctioning optical fibers.

Appropriately, for example, the device according to the invention is particular suited for laser therapy slipped disc treatment because it makes it possible to considerably decrease pain straight away and at the same to intervene on myelin sheath bio-stimulation.

## Claims

1. An improved medical device (2) for laser therapy, comprising:
- a containment casing (4), in which at least two laser sources are housed (16), configured to emit photons having therapeutic action, which have mutually different wavelengths and which are substantially comprised between 550 nm and 1075 nm, and with emission power substantially comprised between 500 mW and 15 W,
- at least two first optical fibers (18), one for each laser source (16), which are housed in said containment casing (4) and in which the photons emitted by the corresponding laser source (16) are sent,
- a single second optical fiber (22) which, at least in part, is external to said containment casing (4),
- a mechanical connection (24) for connecting the output terminals (19) of all said at least two first optical fibers (18) to an input end (21) of said second optical fiber (22) so that the photons output from all said at least two first optical fibers (18) enter into said optical fiber (22),
- an applicator handpiece (26), which is associated with the other end of said second optical fiber (22) and is provided with an opening (30), which communicates with said second optical fiber (22) to let out the photons emitted by said laser sources (16) at the tissue to be treated,
- a control and processing unit (38) configured to control the activation/deactivation of said at least two laser sources (16) so that they emit said photons simultaneously and/or in sequence,
- a user interface (10) for managing the device (2).
and wherein:
- said mechanical connection (24) is configured so that, in its interior, the input end (21) of the second optical fiber (22) faces and/or is aligned with the output terminals (19) of all said first optical fibers (18),
- the cross-section area of the second optical fiber (22) either substantially corresponds with or is preferably greater than the sum of the cross-section areas of all the first optical fibers (18).

2. A device according to claim 1, **characterized in that** said mechanical connection (24) removably connects the output terminals (19) of all said first optical fibers (18) with the input end (21) of the second optical fiber (22).

3. A device according to one or more of the preceding claims, **characterized in that** said mechanical connection (24) is configured so that, inside, the input end (21) of the second optical fiber (22) is either in contact with or in close proximity to the output terminals (19) of all said first optical fibers (18).

4. A device according to one or more of the preceding claims, **characterized in that** said mechanical connection (24) comprises two portions (70, 80), which are mutually separable and couplable by means of joining means (71, 81), the output ends (19) of said first optical fibers (18) being fixed to one (80) of said portions, while the input end (21) of said second optical fiber (22) being fixed to the other portion (70).

5. A device according to one or more of the preceding claims, **characterized in that** said two portions (70, 80) comprise snap-fitting joining means (71, 81).

6. A device according to one or more of the preceding claims, **characterized in that** the output terminals (19) of said first optical fibers (18) are blocked in a tubular leg (81) provided with a female cavity (82) for stably yet removably inserting a protruding male head (71) within which the input end (21) of said second optical fiber (22) is fixed.

7. A device according to one or more of the preceding claims, **characterized in that** it comprises at least two of the following laser sources (16):
- a first laser source, which emits photons at about 580-680 nm with power of about 100 mW - 15 W,
- a second laser source, which emits photons at about 750-795 nm with power of about 500 mW - 15 W,
- a third laser source, which emits photons at about 800-850 nm with power of about 500 mW - 15 W,
- a fourth laser source, which emits photons at about 915-975 nm with power of about 500 mW - 15 W, and
- a fifth laser source, which emits photons at about 1064 nm with power of about 500 mW-15 W.

8. A device according to one or more of the preceding claims, **characterized in that** said control and processing unit (38) is configured to interdependently control each of said at least two laser sources (16) by sending a square wave signal with frequency substantially comprised between 1 and 20000 Hz and duty cycle of at least 50%.

9. A device according to one or more of the preceding claims, **characterized in that** said at least two first optical fibers (18), which are housed in said containment casing (4) and which are each associated with a corresponding laser source (16), are made of silicone and/or **in that** said single second optical fiber (22), which is at least in part external to said containment casing (4), is made using the liquid phase method.

10. A device according to one or more of the preceding claims, **characterized in that** it comprises a cooling system (46, 48, 50), which is housed inside said containment casing (4) and is configured and controlled by said control and processing unit (38) to ensure that the desired and appropriate working temperatures are present in the containment casing (4).

11. A device according to one or more of the preceding claims, **characterized in that** it comprises means (52) for detecting whether the second optical fiber (22) is correctly inserted in said mechanical connection (24) and **in that** said means are functionally connected to said control and processing unit (38) to automatically control the deactivation of said at least two laser sources (16) in case of incorrect insertion of said single fiber (22) in said connection (24).

12. A device according to one or more of the preceding claims, **characterized in that** it comprises a connection (44) with power supply of an external network and/or a battery (54) which is activated by said control and processing unit (38) when an interruption of the network power supply is detected.

13. A device according to one or more of the preceding claims, **characterized in that** said containment casing (4) is made of biocompatible material and is associated with means (6, 8) for easily moving said device within said working space.

## Patentansprüche

1. Verbesserte medizinische Vorrichtung (2) zur Lasertherapie, umfassend:
- ein Aufnahmegehäuse (4), in dem mindestens zwei Laserquellen (16) untergebracht sind, die dazu konfiguriert sind, Photonen mit einer therapeutischen Wirkung zu emittieren, die voneinander verschiedene Wellenlängen aufweisen und die im Wesentlichen zwischen 550 nm und 1075 nm enthalten sind, und wobei die Emissionsleistung im Wesentlichen zwischen 500 mW und 15 W enthalten ist,
- mindestens zwei erste Lichtwellenleiter (18), einer für jede Laserquelle (16), die in dem Aufnahmegehäuse (4) untergebracht sind und in denen die durch die entsprechende Laserquelle (16) emittierten Photonen gesendet werden,
- einen einzelnen zweiten Lichtwellenleiter (22), der sich, mindestens teilweise, außerhalb des Aufnahmegehäuses (4) befindet,
- eine mechanische Verbindung (24) zum Verbinden der Ausgangsanschlüsse (19) von allen der mindestens zwei ersten Lichtwellenleiter (18) mit einem Eingangsende (21) des zweiten Lichtwellenleiters (22), sodass die von allen der mindestens zwei ersten Lichtwellenleiter (18) ausgegebenen Photonen in den Lichtwellenleiter (22) eintreten,
- ein Applikatorhandstück (26), das mit dem anderen Ende des zweiten Lichtwellenleiters (22) verbunden ist und mit einer Öffnung (30) versehen ist, die mit dem zweiten Lichtwellenleiter (22) kommuniziert, um die durch die Laserquellen (16) emittierten Photonen an dem zu behandelnden Gewebe herauszulassen,
- eine Steuer- und Verarbeitungseinheit (38), die dazu konfiguriert ist, die Aktivierung/Deaktivierung der mindestens zwei Laserquellen (16) zu steuern, sodass sie die Photonen gleichzeitig und/oder der Reihe nach emittieren,
- eine Benutzeroberfläche (10) zum Verwalten der Vorrichtung (2),
und wobei:
- die mechanische Verbindung (24) so konfiguriert ist, dass, in ihrem Inneren, das Eingangsende (21) des zweiten Lichtwellenleiters (22) den Ausgangsanschlüssen (19) von allen der ersten Lichtwellenleiter (18) zugewandt ist und/oder an diesen ausgerichtet ist,
- die Querschnittsfläche des zweiten Lichtwellenleiters (22) der Summe der Querschnittsflächen von allen der ersten Lichtwellenleiter (18) entweder im Wesentlichen entspricht oder vorzugsweise größer als diese ist.

2. Vorrichtung nach Anspruch 1, **dadurch gekennzeichnet, dass** die mechanische Verbindung (24) die Ausgangsanschlüsse (19) von allen der ersten Lichtwellenleiter (18) entfernbar mit dem Eingangsende (21) des zweiten Lichtwellenleiters (22) verbindet.

3. Vorrichtung nach einem oder mehreren der vorhergehenden Ansprüche, **dadurch gekennzeichnet, dass** die mechanische Verbindung (24) so konfiguriert ist, dass, innen, das Eingangsende (21) des zweiten Lichtwellenleiters (22) entweder in Kontakt mit oder in unmittelbare Nähe zu den Ausgangsanschlüssen (19) von allen der ersten Lichtwellenleiter (18) ist.

4. Vorrichtung nach einem oder mehreren der vorhergehenden Ansprüche, **dadurch gekennzeichnet, dass** die mechanische Verbindung (24) zwei Abschnitte (70, 80) umfasst, die mittels einem Verbindungsmittel (71, 81) voneinander trennbar oder miteinander koppelbar sind, wobei die Ausgangsenden (19) der ersten Lichtwellenleiter (18) an einem (80) der Abschnitte befestigt sind, während das Eingangsende (21) des zweiten Lichtwellenleiters (22) an dem anderen Abschnitt (70) befestigt ist.

5. Vorrichtung nach einem oder mehreren der vorhergehenden Ansprüche, **dadurch gekennzeichnet, dass** die zwei Abschnitte (70, 80) ein Schnappverbindungsmittel (71, 81) umfassen.

6. Vorrichtung nach einem oder mehreren der vorhergehenden Ansprüche, **dadurch gekennzeichnet, dass** die Ausgangsanschlüsse (19) der ersten Lichtwellenleiter (18) in einem röhrenförmigen Bein (81) blockiert sind, das mit einem weiblichen Hohlraum (82) versehen ist, um einen vorstehenden männlichen Kopf (71), in dem das Eingangsende (21) des zweiten Lichtwellenleiters (22) befestigt ist, stabil aber entfernbar einzuführen.

7. Vorrichtung nach einem oder mehreren der vorhergehenden Ansprüche, **dadurch gekennzeichnet, dass** sie mindestens zwei der folgenden Laserquellen (16) umfasst:
- eine erste Laserquelle, die Photonen mit ungefähr 580-680 nm mit einer Leistung von ungefähr 100 mW bis 15 W emittiert,
- eine zweite Laserquelle, die Photonen mit ungefähr 750-795 nm mit einer Leistung von ungefähr 500 mW bis 15 W emittiert,
- eine dritte Laserquelle, die Photonen mit ungefähr 800-850 nm mit einer Leistung von ungefähr 500 mW bis 15 W emittiert,
- eine vierte Laserquelle, die Photonen mit ungefähr 915-975 nm mit einer Leistung von ungefähr 500 mW bis 15 W emittiert, und
- eine fünfte Laserquelle, die Photonen mit ungefähr 1064 nm mit einer Leistung von ungefähr 500 mW bis 15 W emittiert.

8. Vorrichtung nach einem oder mehreren der vorhergehenden Ansprüche, **dadurch gekennzeichnet, dass** die Steuer- und Verarbeitungseinheit (38) dazu konfiguriert ist, jede der mindestens zwei Laserquellen (16) voneinander abhängig zu steuern, indem sie ein Rechtecksignal mit einer Frequenz, die im Wesentlichen zwischen 1 und 20000 Hz enthalten ist, und einem Tastverhältnis von mindestens 50 % sendet.

9. Vorrichtung nach einem oder mehreren der vorhergehenden Ansprüche, **dadurch gekennzeichnet, dass** die mindestens zwei ersten Lichtwellenleiter (18), die in dem Aufnahmegehäuse (4) untergebracht sind und die jeweils mit einer entsprechenden Laserquelle (16) verbunden sind, aus Silizium bestehen und/oder dass der einzelne zweite Lichtwellenleiter (22), der mindestens teilweise außerhalb des Aufnahmegehäuses (4) untergebracht ist, unter Verwendung des Flüssigphasenverfahrens hergestellt ist.

10. Vorrichtung nach einem oder mehreren der vorhergehenden Ansprüche, **dadurch gekennzeichnet, dass** sie ein Kühlsystem (46, 48, 50) umfasst, das in dem Aufnahmegehäuse (4) untergebracht ist und durch die Steuer- und Verarbeitungseinheit (38) konfiguriert und gesteuert wird, um sicherzustellen, dass die gewünschten und geeigneten Arbeitstemperaturen in dem Aufnahmegehäuse (4) bestehen.

11. Vorrichtung nach einem oder mehreren der vorhergehenden Ansprüche, **dadurch gekennzeichnet, dass** sie ein Mittel (52) zum Erfassen, ob der zweite Lichtwellenleiter (22) korrekt in die mechanische Verbindung (24) eingeführt ist, umfasst, und dass das Mittel funktionell mit der Steuer- und Verarbeitungseinheit (38) verbunden ist, um im Falle eines falschen Einführens des einzelnen Leiters (22) in die Verbindung (24) automatisch die Deaktivierung der mindestens zwei Laserquellen (16) zu steuern.

12. Vorrichtung nach einem oder mehreren der vorhergehenden Ansprüche, **dadurch gekennzeichnet, dass** sie eine Verbindung (44) mit einer Stromversorgung eines externen Netzes und/oder einer Batterie (54) umfasst, die durch die Steuer- und Verarbeitungseinheit (38) aktiviert wird, wenn eine Unterbrechung der Netzstromversorgung erfasst wird.

13. Vorrichtung nach einem oder mehreren der vorhergehenden Ansprüche, **dadurch gekennzeichnet, dass** das Aufnahmegehäuse (4) aus einem biokompatiblen Material besteht und mit einem Mittel (6, 8) zum leichten Bewegen der Vorrichtung in dem Arbeitsraum verbunden ist.

## Revendications

1. Dispositif médical amélioré (2) pour thérapie au laser, comprenant:
- un boîtier de confinement (4), dans laquelle sont logées au moins deux sources laser (16), configurées pour émettre des photons ayant une action thérapeutique, ayant des longueurs d'onde différentes l'une de l'autre et substantiellement comprises entre 550 nm et 1075 nm et avec une puissance d'émission substantiellement compris entre 500 mW et 15 W,
- au moins deux premières fibres optiques (18), une pour chaque source laser (16), qui sont logées dans ledit boîtier de confinement (4) et dans lesquelles sont envoyés les photons émis par la source laser (16) correspondante,
- une deuxième fibre optique (22) qui, au moins en partie, est externe audit boîtier de confinement (4),
- une connexion mécanique (24) pour connecter les extrémités de sortie (19) de toutes lesdites au moins deux premières fibres optiques (18) à une extrémité d'entrée (21) de ladite deuxième fibre optique (22) de sorte que les photons sortants de toutes lesdites au moins deux premières fibres optiques (18) pénètrent dans ladite fibre optique (22),
- une pièce à main d'application (26), qui est associée à l'autre extrémité de ladite deuxième fibre optique (22) et est pourvue d'une ouverture (30), qui communique avec ladite deuxième fibre optique (22) pour laisser sortir les photons émis par lesdites sources laser (16) au niveau du tissu à traiter,
- une unité de commande et de traitement (38) configurée pour contrôler l'activation/désactivation desdites au moins deux sources laser (16) afin qu'elles émettent lesdits photons simultanément et/ou en séquence,
- une interface utilisateur (10) pour gérer le dispositif (2),
et dans lequel:
- ladite connexion mécanique (24) est configurée pour que, à l'intérieur, l'extrémité d'entrée (21) de la deuxième fibre optique (22) soit en face et/ou alignée avec les extrémités de sortie (19) de toutes lesdites premières fibres optiques (18)
- la surface de la section transversale de la deuxième fibre optique (22) soit correspond substantiellement ou est de préférence supérieure à la somme des zones de section transversale de toutes les premières fibres optiques (18).

2. Dispositif selon la revendication 1, **caractérisé en ce que** ladite connexion mécanique (24) relie de manière amovible les extrémités de sortie (19) de toutes lesdites premières fibres optiques (18) à l'extrémité d'entrée (21) de la deuxième fibre optique (22).

3. Dispositif selon une ou plusieurs des revendications précédentes, **caractérisé en ce que** ladite connexion mécanique (24) est configurée pour que, à l'intérieur, l'extrémité d'entrée (21) de la deuxième fibre optique (22) soit en regard et/ou alignée avec les extrémités de sortie (19) de toutes lesdites premières fibres optiques (18).

4. Dispositif selon une ou plusieurs des revendications précédentes, **caractérisé en ce que** ladite connexion mécanique (24) comprend deux parties (70, 80) qui peuvent être séparées et qui peuvent être couplées grâce à de moyens de jonction (71, 81), les extrémités de sortie (19) desdites premières fibres optiques (18) étant fixées à l'une (80) desdites parties, tandis que l'extrémité d'entrée (21) de ladite deuxième fibre optique (22) étant fixée à l'autre partie (70).

5. Dispositif selon l'une ou plusieurs des revendications précédentes, **caractérisé en ce que** lesdites deux parties (70, 80) comprennent des moyens de jonction par encliquetage (71, 81).

6. Dispositif selon l'une ou plusieurs des revendications précédentes, **caractérisé en ce que** les extrémités de sortie (19) desdites premières fibres optiques (18) sont bloquées dans une branche tubulaire (81) munie d'une cavité femelle (82) pour permettre une insertion stable et cependant de manière amovible d'une tête mâle saillante (71) dans laquelle est fixée l'extrémité d'entrée (21) de ladite deuxième fibre optique (22).

7. Dispositif selon l'une ou plusieurs des revendications précédentes, **caractérisé en ce qu'**il comprend au moins deux des sources laser (16) suivantes:
- une première source laser émettant des photons à environ 580-680 nm avec une puissance d'environ 100 mW à 15 W,
- une deuxième source laser émettant des photons à environ 750-795 nm avec une puissance d'environ 500 mW - 15 W,
- une troisième source laser émettant des photons à environ 800-850 nm avec une puissance d'environ 500 mW - 15 W,
- une quatrième source laser, émettant des photons à environ 915-975 nm avec une puissance d'environ 500 mW - 15 W, et
- une cinquième source laser, qui émet des photons à environ 1064 nm avec une puissance d'environ 500 mW-15 W.

8. Dispositif selon l'une ou plusieurs des revendications précédentes, **caractérisé en ce que** ladite unité de commande et de traitement (38) est configurée pour commander de manière interdépendante chacune desdites au moins deux sources laser (16) en envoyant un signal carré avec une fréquence substantiellement compris entre 1 et 20000 Hz et un rapport cyclique d'au moins 50%.

9. Dispositif selon une ou plusieurs des revendications précédentes, **caractérisé en ce que** lesdites au moins deux premières fibres optiques (18), qui sont logées dans ledit boîtier de confinement (4) et qui sont chacune associées à une source laser correspondante (16), sont en silicone et/ou **en ce que** ladite deuxième fibre optique (22), qui est au moins en partie extérieure à ladite boîtier de confinement (4), est réalisée selon le procédé en phase liquide.

10. Dispositif selon une ou plusieurs des revendications précédentes, **caractérisé en ce qu'**il comprend un système de refroidissement (46, 48, 50) qui est logé à l'intérieur dudit boîtier de confinement (4) et qui est configuré et contrôlé par ladite unité de commande et de traitement (38) pour s'assurer que les températures de travail souhaitées et appropriées sont atteintes dans boîtier de confinement (4).

11. Dispositif selon une ou plusieurs des revendications précédentes, **caractérisé en ce qu'**il comprend des moyens (52) pour détecter si la deuxième fibre optique (22) est correctement insérée dans ladite connexion mécanique (24) et **en ce que** lesdits moyens sont fonctionnellement connecté à ladite unité de commande et de traitement (38) pour commander automatiquement la désactivation desdites au moins deux sources laser (16) en cas d'insertion incorrecte de ladite fibre optique (22) dans ladite connexion mécanique (24).

12. Dispositif selon l'une ou plusieurs des revendications précédentes, **caractérisé en ce qu'**il comprend une connexion (44) à l'alimentation d'un réseau externe et/ou d'une batterie (54) activée par ladite unité de commande et de traitement (38) lorsqu'une interruption de l'alimentation réseau est détectée.

13. Dispositif selon une ou plusieurs des revendications précédentes, **caractérisé en ce que** ledit boîtier de confinement (4) est constitué d'un matériau biocompatible et est associé à des moyens (6, 8) pour déplacer facilement ledit dispositif dans ledit espace de travail.
